# EUROPEAN PATENT SPECIFICATION

(11) **EP 2 914 124 B1**
(45) Date of publication and mention of the grant of the patent: **25.01.2017**
(21) Application number: 13803287.5
(22) Date of filing: 01.11.2013
(51) Int. Cl.: A23K 50/80, A23K 20/00, A61P 9/10

(54) **FEED COMPOSITION FOR FISH**
FUTTERMITTEL FÜR FISCH
ALIMENT POUR POISSONS

(30) Priority: 01.11.2012 NO 20121283
(43) Date of publication of application: 09.09.2015
(73) Proprietor: Ewos Innovation AS, 4335 Dirdal (NO)
(72) Inventor: VECINO, José, Luis, González, N-4326 Sandnes (NO); WADSWORTH, Simon, N-9415 Harstad (NO)
(74) Representative: Acapo AS
(86) International application number: PCT/NO2013/000050
(87) International publication number: WO 2014/070020

(56) References cited:
- WO-A2-2009/108067
- WO-A2-2011/031166
- US-A- 4 960 795
- HAUGLAND OYVIND ET AL: "Cardiomyopathy syndrome of atlantic salmon (Salmo salar L.) is caused by a double-stranded RNA virus of the Totiviridae family", JOURNAL OF VIROLOGY, THE AMERICAN SOCIETY FOR MICROBIOLOGY, US, vol. 85, no. 11, 1 June 2011 (2011-06-01), pages 5275-5286, XP009150996, ISSN: 0022-538X
- MENOYO ET AL: "Impact of n-3 fatty acid chain length and n-3/n-6 ratio in Atlantic salmon (Salmo salar) diets", AQUACULTURE, ELSEVIER, AMSTERDAM, NL, vol. 267, no. 1-4, 14 June 2007 (2007-06-14), pages 248-259, XP022116568, ISSN: 0044-8486, DOI: 10.1016/J.AQUACULTURE.2007.02.031
- LAURA MARTINEZ-RUBIO ET AL: "Functional Feeds Reduce Heart Inflammation and Pathology in Atlantic Salmon (Salmo salar L.) following Experimental Challenge with Atlantic Salmon Reovirus (ASRV)", PLOS ONE, vol. 7, no. 11, 30 November 2012 (2012-11-30), page e40266, XP055099990, DOI: 10.1371/journal.pone.0040266

## Description

### Field of the invention

The present invention relates to a feed composition for fish comprising conventional feed ingredients such as proteins, lipids, vitamins, carbohydrates and minerals, for use in the prevention and/or treatment of Cardiomyopathy syndrome (CMS), Hepatic stenosis associated with CMS and/or to diseases caused by the Piscine Myocarditis Virus (PMCV).

### Background of the invention

Cardiomyopathy syndrome (CMS) is a severe cardiac disease of farmed Atlantic salmon (Salmo salar) recently associated with a double-stranded RNA virus termed piscine myocarditis virus (PMCV). The disease was first described and diagnosed in Norway in 1985, and has since also been diagnosed in Scotland, the Faroe Islands and, possibly also Canada. Annual economic losses due to CMS are huge, and this disease has affected 75 to 85 per cent of Norwegian salmon farms each year over the last decade.

Further information can be found in e.g., Haugland et al, Journal of Virology 2011; 85 (11): 5275-5286.

Histopathologically, CMS is characterized by severe inflammation and necrosis of the spongy myocardium of the atrium and ventricle, but liver may also be affected due to the circulatory disturbance associated with the heart lesions. Early stages of the disease have been reported in adult Atlantic salmon around 14 to 18 months after transfer to seawater. Mortality is usually moderate although, as this is a chronic disease developing over a period of several months, cumulative mortality can be relevant.

Clinical signs of CMS or infection by PMCV are typically severe inflammation and degeneration of the spongious part of the myocardium (ventricle) and with similar changes in the atrium. Myocyte degeneration and inflammatory changes are not frequently seen in the compact layer of the heart and always occur later than changes of the spongious parts. Circulatory disturbance with multifocal liver steatosis and necrosis may also occur.

The UK patent application GB2452363 describes an inactivated specific deposited virus that can be used in a vaccine against CMS.

Presently, however, the lack of commercial vaccines to CMS makes the use of alternative therapies crucial, and it is an object of the present invention to provide alternative solutions for the prevention and/or treatment of CMS, liver steatosis and diseases caused by PMCV. Further, it is an object of the present invention to provide feed compositions that can alleviate, inhibit or treat CMS, liver steatosis and diseases caused by PMCV. Such feed compositions is a better alternative than vaccines for the prevention and/or treatment of CMS. Such feed compositions are normally termed functional feeds, and are defined as high-quality feeds that, beyond their nutritional composition, are formulated with health promoting features that could be beneficial in supporting disease resistance and mitigation of clinical disease symptoms. Thus clinical nutrition enables a shift away from vaccines, chemotherapeutic and antibiotic treatments, lowering the costs of disease treatment and management.

In this context, feed compositions for the prevention or the reduction of the symptoms caused by the Heart and Skeletal Muscle Inflammation (HSMI) disease in fish are known from WO 2011/031166 and WO 2009/108067.

Without being bound by theory, we believe that an improved fatty acid profile, i.e. an increased level of n-3 fatty acids in key tissues such as the heart may prevent and/or treat CMC, liver steatosis and diseases caused by PMCV. Further, we believe that reduced digestible energy and increased digestible protein will enhance the effects provided by the improved fatty acid profile.

### Summary of the invention

The present invention relates to a feed composition for fish for the prevention and/or treatment of Cardiomyopathy syndrome (CMS), liver steatosis or diseases caused by the Piscine Myocarditis Virus (PMCV) comprising conventional feed ingredients such as proteins, lipids, vitamins, carbohydrates and minerals, characterized in that the feed comprises fatty acids and that more than 20 % of the total fatty acids are n-3 fatty acids.

In a preferred embodiment is more than 25% of the total fatty acids are n-3 fatty acids, and preferable more than 27% of the total fatty acids are n-3 fatty acids, preferable from 27 to 28%, more preferable from 27.5 to 27.8 %, of the total amount of fatty acids in the feed.

I a preferred embodiment is the content of eicosapentaenoic acid 20:5 n-3 (EPA) more than about 7 % of the total fatty acids.

In a preferred embodiment is the content of eicosapentaenoic acid 20:5 n-3 (EPA) more than about 8 % of the total fatty acids, more preferable more than 9 %, more preferable more than 10 %, more preferable more than 11 %, more preferable more than 12 %, and most preferable more than 13%, more preferable from 13 to 14 %, preferable from 13.4 to 14.7%.

In a preferred embodiment account the proteins for more than 50 % (by weight) and the lipids account for less than 20 % (by weight, preferable about 18% of the feed composition.

In a preferred embodiment is the protein:lipid ratio (w/w) in the feed higher than 2, preferably higher than 2.7, preferable at least2.8, and more preferable at least 2.9.

In a preferred embodiment is the content of n-6 fatty acids less than 8 % of the total fatty acids, preferably in the range of 6-8%, most preferably about 7%.

In a preferred embodiment is the ratio of n-3 fatty acids to n-6 fatty acids higher than 1.6, more preferable higher than 2.0, more preferable higher than 3.0, and most preferable in the range of 3.9 - 4.0.

In a preferred embodiment is a portion of said lipids South American Fish oil.

In a preferred embodiment comprises the composition hydrolysed phospholipids.

In a preferred embodiment comprises the composition krill meal.

In a preferred embodiment is the amount of n-3 fatty acids is is substantially as indicated for CMS or CMS2 in table 1 or 2.

In a preferred embodiment is the amount of eicosapentaenoic acid 20:5 n-3 (EPA)is is substantially as indicated for CMS or CMS2 in table 1 or 2.

In a preferred embodiment is the composition substantially as indicated for CMS or CMS2 in table 1 or 2.

In a preferred embodiment is the fish a Salmonid, preferable Atlantic salmon (Salmo salar).

Preferable, the feed composition is for use in the prevention and/or treatment of Cardiomyopathy syndrome (CMS) in salmonids.

Preferable, the feed composition is for use in the prevention and/or treatment of liver steatosis in salmonids.

Preferable, the feed composition is for use in the prevention and/or treatment of diseases caused by the Piscine Myocarditis Virus (PMCV) is salmonids.

The feed composition according to any of above indicated features, i.e. the features claimed in the claims 1-11, may be used in the manufacturing of a pharmaceutical composition and/or nutritional composition and/or functional feed for the prevention and/or treatment of Cardiomyopathy syndrome (CMS) or diseases caused by the Piscine Myocarditis Virus (PMCV) .

In a preferred embodiment is the fish a salmonid, flat fish or any other fish species suitable for aquaculture, preferable the species Atlantic salmon (Salmo salar).

In a preferred embodiment is the composition fed to a fish that is susceptible to Cardiomyopathy syndrome (CMS) or diseases caused by the Piscine Myocarditis Virus (PMCV) in a period previous to the challenge by an infection, during the infection or after the fish has been infected.

In a preferred embodiment is the composition fed for a period of at least 8 weeks pre infection, more preferably of 10-16 weeks, most preferably of 16-20 weeks and 8-20 weeks post infection.

In a preferred embodiment is the fish a Salmonid, preferable Atlantic salmon (Salmo salar). A preferred embodiment relates to the prevention and/or treatment of Cardiomyopathy syndrome (CMS) in salmonids.

A preferred embodiment relates to the prevention and/or treatment of liver steatosis in salmonids.

A preferred embodiment relates to the prevention and/or treatment of diseases caused by the Piscine Myocarditis Virus (PMCV) is salmonids.

Further aspects of the invention relate to a feed composition or a feeding regime for a fish providing;
- Reduced digestible energy
- Increased digestible protein
- Improved fatty acid profile (preferable EPA, DHA). These fatty acids become functional when they are in polar form and present in the cell membranes of key tissues such as the heart
- Increased phospholipid / reduced triglyceride forms
- Inclusion of functional ingredients such as nucleotides.

### Description of the drawings

Embodiments of the invention will now be described, by the way of examples with reference to the following diagrams, wherein

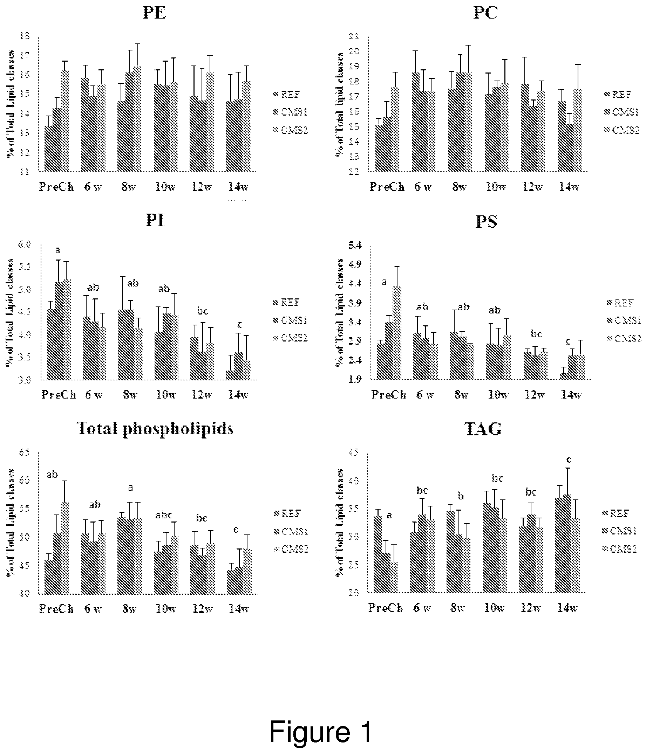

Figure 1 shows the proportions of lipid classes in total lipid of heart tissue from fish fed the reference (REF) and functional (CMS 1 and CMS2) feeds at different times before (PreCh) and after (6, 8, 10, 12 and 14 weeks) infection with PMCV. PC, phosphatidylcholine; PE, phosphatidylethanolamine; PI, phosphatidylinositol; PS, phosphatidylserine; TAG, triacylglycerol.

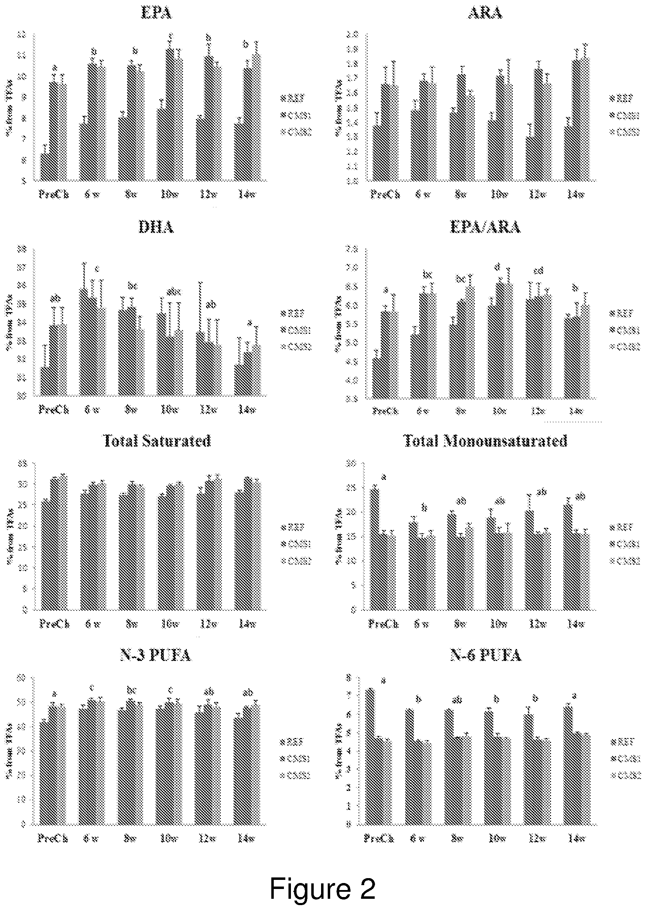

Figure 2 shows the fatty acid compositions (percentage of total fatty acids) of total phospholipids of heart tissue from fish fed the reference (REF) and functional (CMS 1 and CMS2) feeds at different times before (PreCh) and after (6, 8, 10, 12 and 14 weeks) infection with PMCV. ARA, arachidonic acid; DHA, docosahexaenoic acid (DHA); EPA, eicosapentaenoic acid; PUFA, polyunsaturated fatty acid.

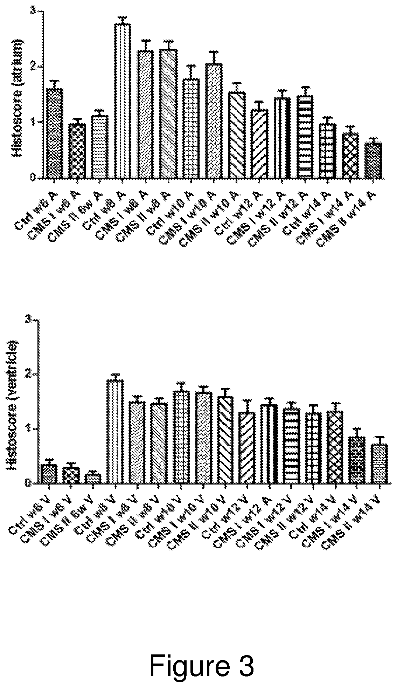

Figure 3 shows the average histoscores (± SEM) of atrium and ventricle in the different dietary treatment groups at 6-, 8-, 10-, 12- and 14-weeks post-challenge

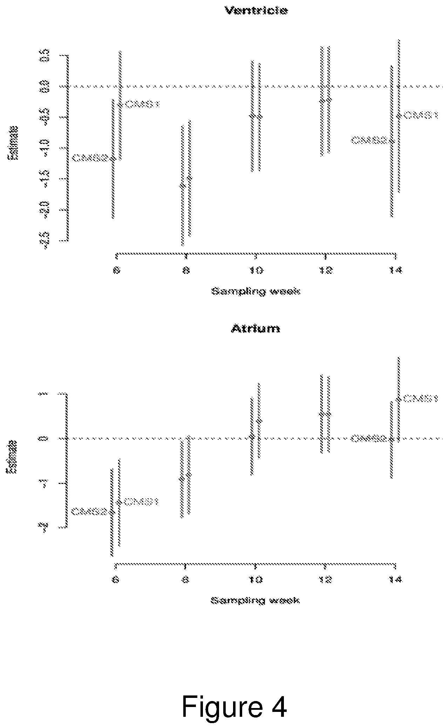

Figure 4 shows the statistical analysis of the atrium and ventriculum histoscores. Estimated effects of CMS 1 and CMS2 diets in comparison to the REF diet by sampling weeks. Negative estimates mean there are lower scores and positive that there are higher scores than for the REF dietary group. Error bars denote approximate 95 % confidence limits.

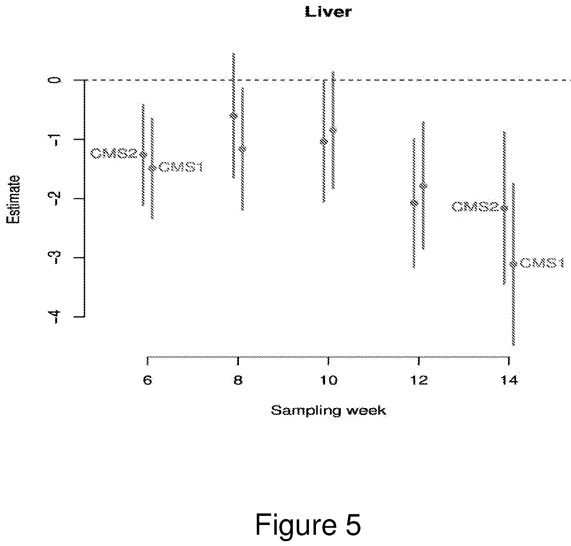

Figure 5 shows the statistical analysis of liver histoscores. Estimated effect of CMS1 and CMS2 diets in comparison to the REF diet by sampling weeks. Negative estimates means there are lower scores and positive that there are higher scores than for the REF dietary group. Error bars denote approximate 95 % confidence limits.

### Description of embodiments of the invention

### Experimental section

### Experimental feeds and fish

Three fishmeal-based diets were formulated and manufactured by EWOS Innovation (Dirdal, Norway) (Table 1). The formulations were based on small-scale commercial screening trials (unpublished). The reference diet (REF) was essentially a standard, commercial formulation with 31 % lipid with the added oil being a blend of Northern hemisphere Fish oil (FO) and rapeseed oil. The two functional feeds (termed CMS 1 and CMS2) both contained high levels of the n-3 fatty acid EPA (almost 4%) and an n-3/n-6 PUFA ratio around 4, in comparison to the REF feed (< 4 % EPA and an n-3/n-6 ratio of 1.4). The two functional feeds are termed CMS 1 and CMS2.

The two functional feeds also contained a lower level of lipid (18 %) that was balanced by increased protein, provided by fishmeal and krill meal.

The only major difference between the functional feeds was that CMS 1 was supplemented with additional histidine.

A total of 675 Atlantic salmon (Salmo salar L.), SalmonBreed IPN svak strain (average weight ca.150 g), were distributed into nine tanks (1 m³) at the VESO facility, Vikan, Norway and fed one of the three feeds (3 tanks of 75 fish each per dietary treatment; 225 fish in total) for a period of 8 weeks prior to being transferred to the challenge tanks. Feeding ration was a maximum of 2 %. Seawater/ brackish water (ca. 25 ‰-35 ‰) delivery was flow-through, being sufficient to maintain oxygen-satiation in effluent water > 70 %. Water temperature was maintained at 12 ± 1 °C, and a photoperiod 24:0 h light/dark regime was followed. Stocking density of the tanks was a maximum of 60 Kg/m³.

**Table 1.**

| Formulation composition (percentage) of the reference (REF) and functional (CMS 1 and CMS2) feeds | | | |
|---|---|---|---|
| Component¹ | REF | CMS1 | CMS2 |
| Crude protein | 42.2 | 53.4 | 53.4 |
| Moisture | 6.5 | 6.5 | 6.5 |
| Fat | 31.0 | 18.0 | 18.0 |
| Ash | 6.2 | 7.7 | 7.7 |
| Histidine (synthetic) | | 4.0 | |
| EPA (% total fatty acids) | 3.7 | 13.7 | 13.4 |
| n-3 (% of total fatty acids) | 13.9 | 27.5 | 27.8 |
| Digestible energy (MJ/kg) | 20.8 | 18.9 | 18.9 |

**Table 2.**

| Fatty acid compositions (percentage of total fatty acids) of the reference (REF) and functional (CMS1 and CMS2) feeds. | | | |
|---|---|---|---|
| Fatty acid | REF | CMS1 | CMS2 |
| Saturated | 16.5 | 26.6 | 25.8 |
| Mounsaturated | 59.1 | 36.4 | 36.1 |
| 18:2n-6 | 8.8 | 4.9 | 5.6 |
| 20:3n-6 | 0.2 | 0.3 | 0.3 |
| 20:4n-6 | 0.2 | 0.6 | 0.7 |
| n-6 PUFA | 9.8 | 6.9 | 7.2 |
| 18:3n-3 | 3.3 | 1.3 | 1.3 |
| 20:5n-3 | 3.7 | 13.7 | 13.4 |
| 22:6n-3 | 5.0 | 8.4 | 8.4 |
| n-3 PUFA | 13.9 | 27.5 | 27.8 |
| PUFA | 24.3 | 37.0 | 38.1 |
| n-3 PUFA/n-6 | | | |
| PUFA | 1.4 | 4.0 | 3.9 |
| EPA/ARA | 19.4 | 21.4 | 20.2 |

### PMCV Challenge

After the pre-feeding period, the fish (non-vaccinated and HSMI-free) were transferred to challenge tanks (1 m³) with the same rearing conditions as described above. Of the available salmon, a total of 576 fish, initial average weight 141.3 g (± 3.30 g, standard error), were distributed into 12 tanks (4 tanks per dietary treatment with 48 fish/tank). The fish were acclimated for 2 weeks prior to challenge and were fed with the same diets during the acclimation period and throughout the period of the challenge (14 weeks) that they were fed prior to transfer. No previous diseases were described.

For challenge, the fish were sedated using Aqui-S at final concentration of 5 mg/L of isoeugenol, followed by anaesthesia in benzocaine (20 %, Benzoak^{®}) using a final concentration of 30 ml/L. Two fish from each pre-challenge tank, 18 in total (n = 6 per dietary treatment) were collected prior to challenge (0 time-point) and then 576 fish (as described above) were challenged by intramuscular injection of 0.1 ml PMCV inoculum on each side of the fish in the lateral muscle tissue beneath the dorsal fin (total, 0.2 ml per fish).

Production of the PMCV inoculum has been described previously. Briefly, the virus was originally isolated from heart tissue homogenate collected from a clinical outbreak of CMS and filtered through a 0.22-µm filter. Challenge of Atlantic salmon was performed using supernatant of GF-1 cell culture in which the virus was inoculated.

### Sampling

Ten fish from each tank (total of 40 per dietary treatment) were sampled at 6-, 8-, 10-, 12-and 14-weeks post-challenge. Fish were anaesthetized as above and killed by a blow to the head and liver and heart tissue collected for analyses. Liver tissue and a portion of each heart were transferred to 10% buffered formalin for histological analyses. Of the remaining portions of heart tissue, half were immediately transferred to RNAlater^{®} (following manufacturers protocol) and stored at -20 °C prior to molecular analyses (i.e. 5 samples of heart tissue per tank, 20 per dietary treatment). The remaining samples of heart tissue were frozen in liquid N₂ and stored at -80 °C prior to lipid analyses (five per tank, 20 per treatment).

### Growth performance

Growth performance of fish fed the three feeds was assessed using repeated measurements of tank mean weights. Tank mean weights were determined at the start of the trial (day 0), challenge-day (day 92) and then at 6-, 8-, 10-, 12- and 14-weeks post-challenge. A linear mixed-effects model (multilevel model) was fitted between the response (weight) and predictor (diet) by allowing the intercepts and slopes of the time variable (day) to vary from tank to tank to account for the tank level correlations and variability in growth trajectories. The model was estimated with the lmer function in the lme4 package of the R language (R Development Core Team, 2008). All treatment effects were based on posterior simulation (n = 2,500) with 95 % credible intervals. Ninety-five percent credible intervals were interpreted as statistical significant at p = 0.05 % level when the interval did not overlap the reference value in question.

### Histological examinations

The histological changes in heart were assessed as described. In brief, the inflammatory changes were evaluated separately for atrium and ventricle, and to the extent present, inflammatory scores of the epicardium were also recorded. Histological samples were collected to include atrium and ventricle in the same sample. Bulbus arteriosus was also included but was not assessed for histopathological changes. Samples were fixed in 10 % buffered formalin, embedded in paraffin wax and sectioned at 3 - 4 micron and stained with hematoxylin and eosin according to standard methods. All sections were evaluated randomly and without knowledge to which feeding group they belonged (double-blind).

Liver steatosis scores were ranked according to a non-continuous score grade from 0 to 5 (Table 3). Briefly, a score of 0 indicated the formation of vacuoles in the cytoplasm, involving less than 10 % of the hepatocytes and including less than 25 % of the area of the individual hepatocytes. A score of 5 indicated formation of vacuoles in the cytoplasm, involving more than 90 % of the hepatocytes and including more than 80 % of the area of the individual hepatocytes. The initial "continuous" scores were converted to a fewer number of discrete scores (3 - 6 classes depending on the response). Scores were an ordinal response and a multilevel ordinal regression was used to analyse the effects of feeds on the scores. This was achieved with the ordinal package Sweave le processed by LATEX of the R language. Each of the scores was used as the response variable in separate analyses that had feed type (REF, CMS 1, CMS2) as the fixed effect term. Since multiple fish were examined from each replicate tank, a random effect of tank was added to the models.

**Table 3**

| Scoring system for liver steatosis in individual sections. | |
|---|---|
| 0 | formation of vacuoles in the cytoplasm, involving less than 10% of the hepatocytes and including less than 25% of the area of the individual hepatocytes |
| 1 | formation of vacuoles in the cytoplasm, involving less than 25% of the hepatocytes and including less than 25% of the area of the individual hepatocytes |
| 2 | formation of vacuoles in the cytoplasm, involving less than 50% of the hepatocytes and including less than 50% of the area of the individual hepatocytes |
| 3 | formation of vacuoles in the cytoplasm, involving less than 75% of the hepatocytes and including less than 75% of the area of the individual hepatocytes |
| 4 | formation of vacuoles in the cytoplasm, involving less than 90% of the hepatocytes and including less than 80% of the area of the individual hepatocytes |
| 5 | formation of vacuoles in the cytoplasm, involving more than 90% of the hepatocytes and including more than 80% of the area of the individual hepatocytes |

### Lipid analyses

Lipid and fatty acid analyses were performed on the feeds and heart samples from all of the sampling points. Three pre-challenge heart samples per dietary treatment were randomly chosen for lipid analysis. Heart tissue from the post-challenge samples were pooled per tank so that heart tissue of five fish became a pool resulting in 4 pools/treatment/sampling point. Total lipid from each pool was extracted by homogenisation in chloroform/methanol (2:1, by volume) according to the method of Folch et al., and lipid content determined gravimetrically. Total lipid extracts were re-suspended in chloroform/methanol (2:1, v/v) + 0.1 % butylated hydroxytoluent (BHT), at a concentration of 10 mg lipid/ml and stored at -70 °C until analysed.

Phospholipid (PL) fractions were prepared from 0.5 mg of total lipid by thin-layer chromatography. Samples were applied to a 20 x 20 cm silica gel 60 TLC plate (VWR, Lutterworth, Leics, UK), and developed in isohexane/diethyl ether/glacial acetic acid (80:20:1, by vol.). The plate was sprayed lightly with 2, 7, dichlorofluorescein (0.1 %, w/v) in 97 % methanol (v/v) and the PL band on the origin scraped from the plate. Fatty acid methyl esters (FAME) were prepared by acid-catalysed transesterification directly on the silica by incubating in 2 ml of 1 % H₂SO₄ in methanol at 50 °C overnight (Christie, 2003). The samples were neutralised with 2.5 ml of 2 % KHCO₃ and extracted twice with 5 ml isohexane/diethyl ether (1:1, v/v) + BHT and the combined extracts dried under a stream of oxygen-free nitrogen and resuspended in 0.3 ml isohexane prior to fatty acid analysis.

FAME were separated and quantified by GLC (Fisons 8160; Carlo Erba, Milan, Italy) using a 60 m x 0.32 mm x 0.25 µm film thickness capillary column (ZB-WAX; Phenomenex, Macclesfield, Cheshire, UK). Hydrogen was used as the carrier gas at a flow rate of 4.0 ml/min and the temperature programme was from 50 to 150 °C at 40 °C /min then to 195 °C at 2 °C /min and finally to 215 °C at 0.5 °C /min. Individual FAME were identified by comparison with well-characterised in-house standards as well as commercial FAME mixtures (Supelco ™ FAME mix; Sigma-Aldrich Ltd, Gillingham, Dorset, UK).

Tissue and diet lipid class compositions were determined by single-dimension double-development high-performance thin-layer chromatography (HPTLC) and densitometry as described previously (Bell et al., 1993). Significance of differences due to diet and time were determined by two-way ANOVA (p<0.05) using the SPSS 19.0 statistical package (SPSS Inc., Chicago IL, USA).

### Results

### Growth

There were no significant differences overall between diets at the end of the trial (i.e. after 192 days) (data not shown).

### Lipid and fatty acid composition of heart tissue

Total lipid content of heart tissue was similar irrespective of dietary treatment and did not show any significant changes over the course of infection (Table 4).

**Table 4.**

| Lipid contents (percentage of wet weight) of heart tissue of salmon fed the reference (REF) and functional (CMS 1 and CMS2) feeds immediately prior to challenge with PMCV (PreCh) and at different weeks post-challenge (wpc). | | | | | | |
|---|---|---|---|---|---|---|
| | REF | | CMS1 | | CMS 2 | |
| PreCh | 2.8 | ± 0.2 | 3.5 | ± 0.1 | 3.6 | ± 0.2 |
| 6-wpc | 3.3 | ± 0.2 | 3.3 | ± 0.2 | 3.5 | ± 0.2 |
| 8-wpc | 3.8 | ± 0.4 | 3.3 | ± 0.2 | 3.6 | ± 0.6 |
| 10-wpc | 3.8 | ± 0.3 | 3.6 | ± 0.2 | 3.6 | ± 0.3 |
| 12-wpc | 3.8 | ± 0.2 | 3.6 | ± 0.1 | 3.5 | ± 0.1 |
| 14-wpc | 3.8 | ± 0.3 | 3.4 | ± 0.5 | 3.5 | ± 0.4 |

There were no significant differences between time-points.

Levels of total phospholipids (PL) and the major PL classes, phosphatidylcholine (PC) and phosphatidylethanolamine (PE), were significantly higher in heart tissue in fish fed the functional feeds (Figure 1) compared with heart tissue of fish fed the REF diet (2-way ANOVA p-value diet, < 0.05). This was probably a consequence of the levels of triacylglycerols (TAG) being significantly higher in the REF group, with these differences being more obvious at the end of the initial feeding phase prior to infection with PMCV. After PMCV infection, PL levels generally decreased over the course of the infection with this effect being significant for phosphatidylinositol (PI) and phosphatidylserine (PS).

The fatty acid compositions of total PL of heart tissue of fish fed the functional feeds were characterised by lower proportions of monoenes and n-6 PUFA, and higher proportions of saturated fatty acids, n-3 PUFA, LC-PUFA (ARA, EPA and DHA), and EPA/ARA ratio (Figure 2). The two functional feeds presented similar levels in the relative proportions of the important LC-PUFA, ARA, EPA and DHA. Thus, heart tissue phospholipids showed proportions of the LC-PUFA relevant to eicosanoid pathways, and that could therefore potentially influence the immune response (Calder 2009b), that reflected the composition of the diets. The proportions of EPA, ARA and the EPA/ARA ratio were significantly higher in fish fed the functional feeds compared to fish fed the REF diet (P value diet, <0.05). However, levels of DHA were similar between the different dietary groups, which are consistent with our previous study using similar feeds in which levels of DHA were more conserved despite differences in the composition of the diets (Martinez-Rubio et al., 2012b).

Changes in the proportions of the immune and inflammation-related LC-PUFA in heart total PL during the time course of the infection were similar between the three different groups (Figure 2). The changes in ARA post-infection were not significant, but the proportions of EPA and DHA were significantly higher at 6-wpc compared with pre-challenge. After 6-wpc, levels of DHA progressively decreased during the time-course of the infection but proportions of EPA were significantly higher at 10-wpc. As a consequence of the latter, and the changes in ARA levels, the EPA/ARA ratio in heart total PL was also higher in all dietary treatments at 10-wpc. There were no differences in the EPA/ARA ratio in heart PL between fish fed the different diets in the later stages of the infection.

### Heart and liver tissue histopathology

Histological changes were first observed in the atrium at 6-wpc (Figure 3), characterised by focal infiltration of inflammatory cells dominated by lymphocytes. A degenerative process associated with the inflammatory changes was also observed in cardiomyocytes of the atrium. By 8-wpc the averages scores for atrium and ventricle had increased to 4.1 in fish fed the REF diet, and 3.5 for fish fed both functional feeds. The most marked increase in histopathological changes (from 6- to 8-wpc) was found in the ventricle with a 5.6-fold increase in the REF group, and 5.1- and 9.1-fold for the CMS1 and CMS2 dietary groups, respectively. These changes were typified by multifocal infiltration of inflammatory cells, dominated by lymphocytes and macrophages, and concomitant degeneration and necrosis of myocytes. Another typical finding was hyperplasia of endothelial cells in inflamed areas. Inflammatory changes were greatest in the atrium from 6- to 8-wpc and significantly (p < 0.01) higher for all groups at both time points. Both groups of fish fed with the functional diets showed significantly lower histoscores in the atrium (Figure 4) at both 6- and 8-wpc. Lesions in the ventricle were also significantly lower for the group of fish fed with the functional feeds. By 10-wpc, the inflammatory changes and myocyte necrosis had levelled off in all groups, and was not different from 8-wpc. At 12- and 14-wpc there was a moderate decline in inflammatory scores (Figure 3). There were no differences between dietary groups at 10- and 12-wpc in both parts of the heart. At the end of the trial, 14-wpc, lesions in the atrium were not significantly different between groups, although fish fed with the CMS2 have lower histoscores compare with the other groups. In ventricle, fish fed both functional feeds showed lower histoscores although they were not significant (Figure 4).

The liver histology scores, based on the degree of steatosis were read as vacuole-formation in hepatocyte cytoplasm characterised by both micro- and macro-vesicular lesions. There were clear differences between the fish fed the functional feeds and fish fed the REF diet over all the samplings points post-challenge, with the latter group presenting higher micro- and macro-vesicular steatosis. Severity of the liver histopathology (based on the scoring system in Table 3) was significantly higher at the beginning of the infection and greater at the end of the challenge (12- and 14-wpc) in salmon fed the REF diet compared to fish fed the two functional feeds (Figure 5).

### Conclusions

Functional feeds in accordance with the present invention were clearly beneficial for the liver pathology associated with CMS. Fish fed both functional feeds showed lower histoscores over the whole course of the infection compared to fish fed the REF diet.

The potential bioavailability of EPA and ARA, and EPA's proportion relative to ARA was always higher over the time-course of the infection in fish fed the functional feeds compared with fish fed the REF diet.

In addition to effects on fatty acid composition, the PL class composition was also affected by the viral infection, with the main change being the significant decrease in the proportions of PI and PS as the infection progresses.

The functional feeds in accordance with the present invention show a clear delay in the appearance of the histological changes associated with the progress of CMS heart pathology in fish.

As the CMS disease progressed the extent of lesions in the heart increased in both atrium and ventricle with the differences between the fish fed the functional feeds and fish fed the REF diet being most pronounced at 8-wpc. At this time-point, the fish fed the functional feeds, presented significantly lower histoscores in both atrium and ventricle compared with fish fed the REF diet.

### Definitions of terms and abbreviations:

"FA" is the abbreviation used for "fatty acids"
"n-3 fatty acids" are a family of unsaturated fatty acids that have in common a final carbon-carbon double bond in the n-3 position, which is the third bond from the methyl end of the fatty acid. These fatty acids are also known as omega 3 fatty acids.
"n-6 fatty acids" are a family of unsaturated fatty acids that have in common a final carbon-carbon double bond in the n-6 position, which is the sixth bond from the methyl end of the fatty acid. These fatty acids are also known as omega 6 fatty acids.

EPA is eicosapentaenoic acid 20:5 n-3.

ARA is arachidonic acid 20:4 n-6.

By the term "n-3/n-6 ratio" is meant the ratio between fatty acids belonging to the n-3 family and those belonging to the n-6 family in the composition or diet.

"Protein/lipid ratio" means the ratio between proteins and lipids comprised in the composition or diet (each of them related to the weight of the diet).

"CMS" is the abbreviation used for "Cardiomyopathy syndrome".

"PMCV" is the abbreviation for Piscine Myocarditis Virus.

The term "conventional feed ingredients" refers to ingredients which are commonly in fish feeds such as proteins, lipids, carbohydrates, vitamins, minerals, etc. The ingredients can be derived from marine, vegetable, animal by-products and/ or any other relevant sources and can be used in any suitable combination.

## Claims

1. A feed composition for fish for use in the prevention and/or treatment of diseases caused by the Piscine Myocarditis Virus (PMCV), including Cardiomyopathy syndrome (CMS) and liver steatosis caused by PMCV, comprising conventional feed ingredients such as proteins, lipids, vitamins, carbohydrates and minerals, **characterized in that** the feed comprises fatty acids and that more than 20 % of the total fatty acids are n-3 fatty acids.

2. Feed composition for the use according to claim 1, wherein more than 25% of the total fatty acids are n-3 fatty acids, and preferably more than 27% of the total fatty acids are n-3 fatty acids.

3. Feed composition for the use according to claim 1, wherein the amount of n-3 fatty acids are from 27 to 28%, more preferably from 27.5 to 27.8 %, of the total amount of fatty acids in the feed.

4. Feed composition for the use according to any of the preceding claims, wherein the content of eicosapentaenoic acid 20:5 n-3 (EPA) is more than about 7 % of the total fatty acids.

5. Feed composition for the use according to claim 3, wherein the content of eicosapentaenoic acid 20:5 n-3 (EPA) is more than about 8 % of the total fatty acids, more preferably more than 9 %, more preferably more than 10 %, more preferably more than 11 %, more preferably more than 12 %, and most preferably more than 13%.

6. Feed composition for the use according to claim 1, wherein the content of eicosapentaenoic acid 20:5 n-3 (EPA) is from 13 to 14%, preferably from 13.4 to 14.7%.

7. Feed composition for the use according to any of the preceding claims, wherein the proteins account for more than 50 % (by weight) and the lipids account for less than 20 % (by weight, preferably about 18% of the feed composition.

8. Feed composition for the use to any of the preceding claims, wherein the protein:lipid ratio (w/w) in the feed is higher than 2, preferably higher than 2.7, preferably at least 2.8, and more preferably at least 2.9.

9. A feed composition for the use according to any of the previous claim, wherein the content of n-6 fatty acids are less than 8 % of the total fatty acids, preferably in the range of 6-8%, most preferably about 7%.

10. A feed composition for the use according to any of the preceding claims, wherein the ratio of n-3 fatty acids to n-6 fatty acids is higher than 1.6, more preferably higher than 2.0, more preferably higher than 3.0, and most preferably in the range of 3.9-4.0.

11. A feed composition for the use according to any of the previous claims wherein a portion of said lipids is South American Fish oil.

12. A feed composition for the use according to any of the previous claims wherein the composition comprises hydrolysed phospholipids.

13. A feed composition for the use according to any of the previous claims wherein the composition comprises krill meal.

14. Feed composition for the use according to any of the previous claims, wherein said fish is a Salmonid, preferably Atlantic salmon (Salmo salar).

15. Feed composition for the use according to any of the claims 1-14, wherein said use is the prevention and/or treatment of Cardiomyopathy syndrome (CMS) in salmonids.

16. Feed composition for the use according to any of the claims 1-14, wherein said use is the prevention and/or treatment of liver steatosis in salmonids.

17. Feed composition for the use according to any of the claims 1-14, wherein said use is the prevention and/or treatment of diseases caused by the Piscine Myocarditis Virus (PMCV) in salmonids.

## Patentansprüche

1. Futterzusammensetzung für Fische zur Verwendung bei der Vorbeugung und/oder Behandlung von durch das Fischmyokarditisvirus (PMCV) hervorgerufenen Erkrankungen, einschließlich Kardiomyopathie-Syndrom (CMS) und durch PMCV hervorgerufener Lebersteatose, umfassend herkömmliche Futterinhaltsstoffe wie etwa Proteine, Lipide, Vitamine, Kohlenhydrate und Mineralien, **dadurch gekennzeichnet, dass** das Futter Fettsäuren umfasst und dass mehr als 20 % der gesamten Fettsäuren n-3-Fettsäuren sind.

2. Futterzusammensetzung zur Verwendung nach Anspruch 1, wobei mehr als 25 % der gesamten Fettsäuren n-3-Fettsäuren sind und bevorzugt mehr als 27 % der gesamten Fettsäuren n-3-Fettsäuren sind.

3. Futterzusammensetzung zur Verwendung nach Anspruch 1, wobei die Menge von n-3-Fettsäuren von 27 bis 28 %, bevorzugter von 27,5 bis 27,8 % der Gesamtmenge von Fettsäuren in dem Futter beträgt.

4. Futterzusammensetzung zur Verwendung nach einem der vorstehenden Ansprüche, wobei der Gehalt von Eicosapentaensäure 20:5 n-3 (EPA) mehr als etwa 7 % der gesamten Fettsäuren beträgt.

5. Futterzusammensetzung zur Verwendung nach Anspruch 3, wobei der Gehalt von Eicosapentaensäure 20:5 n-3 (EPA) mehr als etwa 8 % der gesamten Fettsäuren, bevorzugter mehr als 9 %, bevorzugter mehr als 10 %, bevorzugter mehr als 11 %, bevorzugter mehr als 12 % und am bevorzugtesten mehr als 13 % beträgt.

6. Futterzusammensetzung zur Verwendung nach Anspruch 1, wobei der Gehalt von Eicosapentaensäure 20:5 n-3 (EPA) von 13 bis 14 %, bevorzugt von 13,4 bis 14,7 % beträgt.

7. Futterzusammensetzung zur Verwendung nach einem der vorstehenden Ansprüche, wobei die Proteine mehr als 50 (Gew.-)% und die Lipide weniger als 20 (Gew.-)%, bevorzugt etwa 18 % der Futterzusammensetzung ausmachen.

8. Futterzusammensetzung zur Verwendung nach einem der vorstehenden Ansprüche, wobei das Protein:Lipid-Verhältnis (Gew./Gew.) in dem Futter größer als 2, bevorzugt größer als 2,7, bevorzugt mindestens 2,8 und bevorzugter mindestens 2,9 ist.

9. Futterzusammensetzung zur Verwendung nach einem der vorstehenden Ansprüche, wobei der Gehalt von n-6-Fettsäuren weniger als 8 % der gesamten Fettsäuren, bevorzugt in dem Bereich von 6-8 %, am bevorzugtesten etwa 7 % beträgt.

10. Futterzusammensetzung zur Verwendung nach einem der vorstehenden Ansprüche, wobei das Verhältnis von n-3-Fettsäuren zu n-6-Fettsäuren größer als 1,6, bevorzugter größer als 2,0, bevorzugter größer als 3,0 und am bevorzugtesten in dem Bereich von 3,9-4,0 ist.

11. Futterzusammensetzung zur Verwendung nach einem der vorstehenden Ansprüche, wobei ein Teil der Lipide südamerikanisches Fischöl ist.

12. Futterzusammensetzung zur Verwendung nach einem der vorstehenden Ansprüche, wobei die Zusammensetzung hydrolysierte Phospholipide umfasst.

13. Futterzusammensetzung zur Verwendung nach einem der vorstehenden Ansprüche, wobei die Zusammensetzung Krillmehl umfasst.

14. Futterzusammensetzung zur Verwendung nach einem der vorstehenden Ansprüche, wobei der Fisch ein Salmonid, bevorzugt atlantischer Lachs (Salmo salar) ist.

15. Futterzusammensetzung zur Verwendung nach einem der Ansprüche 1-14, wobei die Verwendung die Vorbeugung und/oder Behandlung von Kardiomyopathie-Syndrom (CMS) bei Salmoniden ist.

16. Futterzusammensetzung zur Verwendung nach einem der Ansprüche 1-14, wobei die Verwendung die Vorbeugung und/oder Behandlung von Lebersteatose bei Salmoniden ist.

17. Futterzusammensetzung zur Verwendung nach einem der Ansprüche 1-14, wobei die Verwendung die Vorbeugung und/oder Behandlung von durch das Fischmyokarditisvirus (PMCV) hervorgerufenen Erkrankungen bei Salmoniden ist.

## Revendications

1. Composition d'aliment pour poisson pour l'utilisation dans la prévention et/ou le traitement de maladies provoquées par le virus de la myocardite piscicole (PMCV), notamment le syndrome de cardiomyopathie (SCM) et la stéatose hépatique provoquée par le PMCV, comprenant des ingrédients alimentaires conventionnels tels que des protéines, des lipides, des vitamines, des hydrates de carbone et des minéraux, **caractérisée en ce que** l'aliment comprend des acides gras et **en ce que** plus de 20 % des acides gras totaux sont des acides gras n-3.

2. Composition d'aliment pour l'utilisation selon la revendication 1, dans laquelle plus de 25 % des acides gras totaux sont des acides gras n-3, et de préférence plus de 27 % des acides gras totaux sont des acides gras n-3.

3. Composition d'aliment pour l'utilisation selon la revendication 1, dans laquelle la quantité d'acides gras n-3 va de 27 à 28 %, plus préférablement de 27,5 à 27,8 %, de la quantité totale d'acides gras dans l'aliment.

4. Composition d'aliment pour l'utilisation selon l'une quelconque des revendications précédentes, dans laquelle la teneur en acide eicosapentaénoïque 20:5 n-3 (EPA) est supérieure à environ 7 % des acides gras totaux.

5. Composition d'aliment pour l'utilisation selon la revendication 3, dans laquelle la teneur en acide eicosapentaénoïque 20:5 n-3 (EPA) est supérieure à environ 8 % des acides gras totaux, plus préférablement supérieure à 9 %, plus préférablement supérieure à 10 %, plus préférablement supérieure à 11 %, plus préférablement supérieure à 12 % et le plus préférablement supérieure à 13 %.

6. Composition d'aliment pour l'utilisation selon la revendication 1, dans laquelle la teneur en acide eicosapentaénoïque 20:5 n-3 (EPA) va de 13 à 14 %, de préférence de 13,4 à 14,7 %.

7. Composition d'aliment selon l'une quelconque des revendications précédentes, dans laquelle les protéines représentent plus de 50 % (en poids) et les lipides représentent moins de 20 % (en poids), de préférence environ 18 % de la composition d'aliment.

8. Composition d'aliment pour l'utilisation selon l'une quelconque des revendications précédentes, dans laquelle le rapport protéines:lipides (p/l) dans l'aliment est supérieur à 2, de préférence supérieur à 2,7, de préférence d'au moins 2,8, et plus préférablement d'au moins 2,9.

9. Composition d'aliment pour l'utilisation selon l'une quelconque des revendications précédentes, dans laquelle la teneur en acides gras n-6 est inférieure à 8 % des acides gras totaux, de préférence dans la plage de 6 à 8 %, plus préférablement d'environ 7 %.

10. Composition d'aliment pour l'utilisation selon l'une quelconque des revendications précédentes, dans laquelle le rapport des acides gras n-3 aux acides gras n-6 est supérieur à 1,6, plus préférablement supérieur à 2,0, plus préférablement supérieur à 3,0, et le plus préférablement dans la plage de 3,9 à 4,0.

11. Composition d'aliment pour l'utilisation selon l'une quelconque des revendications précédentes dans laquelle une partie desdits lipides est de l'huile de poisson d'Amérique du sud.

12. Composition d'aliment pour l'utilisation selon l'une quelconque des revendications précédentes, la composition comprenant des phospholipides hydrolysés.

13. Composition d'aliment pour l'utilisation selon l'une quelconque des revendications précédentes, la composition comprenant de la farine de krill.

14. Composition d'aliment pour l'utilisation selon l'une quelconque des revendications précédentes, ledit poisson étant un salmonidé, de préférence le saumon de l'Atlantique (Salmo salar).

15. Composition d'aliment pour l'utilisation selon l'une quelconque des revendications 1 à 14, ladite utilisation étant la prévention et/ou le traitement du syndrome de cardiomyopathie (SCM) chez les salmonidés.

16. Composition d'aliment pour l'utilisation selon l'une quelconque des revendications 1 à 14, ladite utilisation étant la prévention et/ou le traitement de la stéatose hépatique chez les salmonidés.

17. Composition d'aliment pour l'utilisation selon l'une quelconque des revendications 1 à 14, ladite utilisation étant la prévention et/ou le traitement de maladies provoquées par le virus de la myocardite piscicole (PMCV) chez les salmonidés.
